# EUROPEAN PATENT APPLICATION

(11) **EP 3 407 361 A1**
(43) Date of publication of application: **28.11.2018**
(21) Application number: 17172787.8
(22) Date of filing: 24.05.2017
(51) Int. Cl.: H01B 1/12, C07D 471/04, C07F 1/08, C07D 213/22, H01G 9/20, H01L 51/00, H01L 51/42

(54) **REDOX MELTS FORMED BY COPPER (I)/(II) COMPLEXES AS CHARGE TRANSFER AND CHARGE STORAGE MATERIALS**

(71) Applicant: Ecole Polytechnique Fédérale de Lausanne (EPFL), 1015 Lausanne (CH)
(72) Inventor: Stojanovic, Marko, 1213 Onex (CH); Zakeeruddin, Shaik Mohammed, 1030 Bussigny (CH); Graetzel, Michael, 1025 St-Sulpice (CH); Hagfeldt, Anders, 1132 Lully (CH); Cao, Yiming, 1004 Lausanne (CH); Freitag, Marina, 756 44 Uppsala (SE)
(74) Representative: Ganguillet, Cyril

(57) **Abstract**

The present invention relates to a redox copper complex melt comprising a metalorganic complex of formula (I)

[Cu (La)ₙ] (I),

wherein n is an integer 2; and

La is a bidentate ligand selected from a ligand according to any one of formulae (1) and (2):

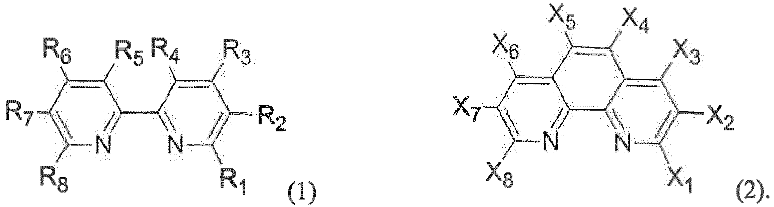

## Description

### Technical Field

The present invention relates to the fields of metal complex melts, of metal complex melts used as redox couples in to optoelectronic and/or electrochemical device comprising metal complex melts, and a method of producing said metal complex melts.

### Background of the Invention

Melts refer to metals or other materials in a liquid condition having a melting point below 100°C. Redox metal complex melts are different from ionic liquids generally referring to salts having an organic component and that have a melting point below 100 °C. Ionic liquids are often based on PF₆⁻, BF₄⁻, [B(CN)₄]⁻, CF3SO₃⁻, [(CF₃SO₂)₂N]⁻ (TFSI), AlCl₃ etc., and various salts of n-alkyl pyridinium halide or organic salts, in particular of pyridinium type, imidazolium type, quarternary ammonium salt type and they are used for catalysis, synthesis, in lithium battery, in fuel cell and solar cell as electrolytes or solvent (WO2009/083901 A1) as well as solvent for metal complexes (US 20040097755 A1).

One of the key components of dye-sensitized solar cell (DSC) is hole conductor (HC) transporting positive charge carriers from the sensitizer to the back contact of the device and regenerating the oxidized dye molecules. Electrolytes containing redox organometallic systems or redox metal complex couples are commonly used as HCs due to their high reliability, good power conversion efficiency (PCE) and their non-corrosiveness and stability compared to the I⁻/I₃⁻ couple. Redox mediators play a major role determining the photocurrent and the photovoltage in DSCs. To maintain the photocurrent, the reduction of oxidized dye by the redox mediator should be significantly faster than the electron back transfer between TiO₂ and the oxidized dye. The driving force for dye regeneration with the redox mediator should be sufficiently low to provide high photovoltages. Some metal complex redox couples do not exhibit a high over-potential for dye regeneration, provide a relatively high or increased open circuit potential (V_{OC}) and have high oxidation potential and/or adjustable oxidation potential.

Among these redox metal complexes or redox mediators or redox shuttle, several copper complexes, e.g. bis(1,10-phenanthroline)copper ([Cu(phen)₂]^{2+/+}), [(-)-sparteine-*N*,*N'*]-(maleonitriledithiolato-*S*,*S'*)copper ([Cu(SP)(mmt)]^{0/-}) and bis(2,9-dimethyl-1,10 - phenanthroline)copper ([Cu(dmp)₂]^{2+/+}) dissolved in a solvent, have been demonstrated as fastest electron-transfer mediators of electrolytes for solar-to-electricity conversion in DSCs. Especially [Cu(dmp)₂]^{2+/+} exhibits the fastest electron self-exchange rate and provides a power conversion efficiency (PCE) of 2.2% at a light intensity of 200 W/m² up to 10% under air mass 1.5 global (AM1.5G) irradiation intensity of 1,000 W/m² with novel organic dye molecules in DSCs. [Cu(dmp)₂]^{2+/+} complexes were recently used as HTMs for over 8% solid-state DSCs (ssDSCs) (Freitag et al., High-efficiency dye-sensitized solar cells with molecular copper dimethyl phenanthroline as solid hole conductor. Energy Environ. Sci. 8, 2634-2637 (2015)), mainly because of the rapid electron self-exchange rate in copper complexes, However, such copper complexes based HTM can form crystalline phases, deteriorating the device performance.

One of the drawbacks of these metal redox complexes is that the formation of crystalline phase when they are in the solid state, what creates a contact problem with the counter electrode and is responsible for the device instability.

The development of new kind of redox metalorganic mediators rending rapid electron self-exchange rate and avoiding the crystallization is required to increase the efficiency and stability of ssDSC and DSC devices, to improve the performance of solar cell and long term stability without optimizing the device by dye co-sensitization e.g., to decrease their fabrication cost and to increase their stability.

The present invention addresses the problems depicted above.

### Summary of the Invention

Surprisingly, the present inventors have found that metal complex melts, namely copper complexes under melt state, provide redox mediators which can be used directly in DSCs. The advantage of the redox melts is that they do not crystallize when they are in the solid phase. The fact that they are either in the form of liquid or in amorphous phase, makes them to provide stable and sustainable device performance.

The inventors also have found that substituting either the organic moiety of the cationic metal complex with alkyl chains comprising alkoxy groups or ether groups, or complexing the cationic metalorganic complex with a counterion or an anion comprising an aryl moiety substituted by alkyl chain comprising alkoxy groups or ether groups, or both allows to obtain a metalorganic complex under melt state.

The present invention provides a redox copper complex melt having a melting point below 100°C and comprising a metalorganic complex of formula (I) : [Cu (La)ₙ] (I), wherein n is an integer 2; and, La is a bidentate ligand selected from a ligand according to any one of formulae (1) and (2): 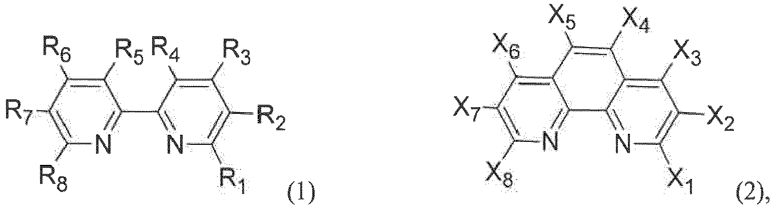 R₁, R₂, R₁, R₂, R₃, R₄, R₅, R₆, R₇, R₈, X₁, X₂, X₃, X₄, X₅, X₆, X₇, and X₈ are independently selected from H, halogen, Cl⁻, Br⁻, I⁻,-NO₂, -OH, -NH₂, -COOH, -CN, -OCN⁻, isocyanate group, sulfonyl group and from substituents comprising 1 to 40 carbons and 0 to 20 heteroatoms and/or groups selected from C1-C25 alkyl group, C4-C20 aryl group, C4-C20 arylalkyl group, C1-C25 alkoxy group, C4-C25 alkoxyalkoxyalkoxyalkyl group, C1-C20 heteroalkyl group, C4-C20 heteroaryl group, C4-C20 heteroarylalkyl group, said heteroatom being selected from N, S, or O, and from -O-, -O-R⁶, -O-R⁶-R⁶,-C(O)-O-R⁶, -C(O)-(NR⁷)-O-R⁶, -C(O)-(NR⁷)-R⁶; with R⁶ being independently selected from C1-C25 alkyl group or 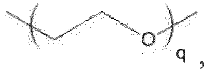 q being an integer from 1 to 20, and with R⁷being selected from H or C1-C16 alkyl group, from -(CH₂)₂-O-, -C(O)-, -C(O)O-, -S-, -S(O)-, SO₂-, -S(O)₂O-, -N=, -P=,-NR⁹-, -PR⁹-, -P(O)(OR⁹)-, -P(O)(OR⁹)O-, -P(O)(NR⁹R⁹)-, -P(O)(NR⁹R⁹)O-,-P(O)(NR⁹R⁹)NR⁹-, -S(O)NR⁹-, and -S(O)₂NR⁹, with R⁹ being independently selected from H, C1-C6 alkyl, C4-C20 aryl group, C4-C20 arylalkyl group and C4-C20 heteroaryl group, wherein the heteroatom is selected from N, S, or O, and said alkyl, arylalkyl and heteroaryl groups being optionally perfluorinated.

In a further aspect, the invention provides an optoelectronic and/or electrochemical device or redox flow batteries comprising at least one or more redox copper complex melt according to the invention.

In another aspect, the invention provides a method for producing a redox copper complex melt comprising a metalorganic complex of formula (I), comprising providing a solution comprising a ligand La selected from formula (1) or from formula (2), and further comprising a salt of copper , exchanging the anion provided by the copper salt by titration with a solution comprising an anion selected from ClO₄⁻, PF₆⁻, BF₄⁻, [B(CN)₄]⁻, CF3SO₃⁻, [(CF₃SO₂)₂N]⁻ (TFSI), [B(C₆H₃(m-CF₃)₂)₄]⁻, [B(C₆F₅)₄]⁻, [B(C₆H₅)₄]⁻, [Al(OC(CF₃)₃)₄]⁻, [CB₁₁H₁₂]⁻, a moiety according to formula (3): 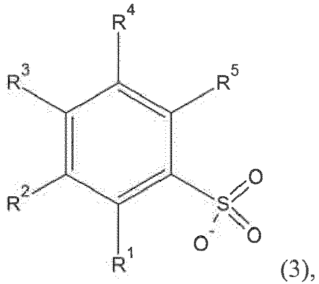 wherein R¹, R², R³, R⁴, R⁵ are independently selected from H and substituents comprising 1 to 40 carbons and 0 to 20 groups selected from C1-C16 alkyl group, C1-C25 alkoxy group, C4-C25 alkoxyalkoxyalkoxyalkyl group, C4-C20 aryl group, C4-C20 arylalkyl group, -O-, -CF₃, -O-R⁶, -O-R⁶-R⁶,-C(O)-O-R⁶, -C(O)-(NR⁷)-OR⁶, -C(O)-(NR⁷)-R⁶, with R⁶ being independently selected from C1-C25 alkyl group or 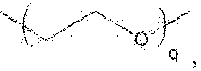 q being an integer from 1 to 20, and with R⁷ being selected from H or C1-C16 alkyl group; or a salt of a moiety of formula (3); and evaporating the titrated crude solution to obtain an melt comprising the redox copper complex melt of the invention.

Further aspects and preferred embodiments of the invention are detailed herein below and in the appended claims. Further features and advantages of the invention will become apparent to the skilled person from the description of the preferred embodiments given below.

### Brief Description of the Drawings

**Figure 1A****:** shows the schematic synthesis of Copper(I) bis(4,4',6,6'-tetramethyl-2,2'-bipyridine) 2-(2,5,8,11-tetraoxadodecanoyl)benzenesulfonate. **Figure 1B** shows the schematic synthesis of Copper(II) bis (4,4',6,6'-tetramethyl-2,2'-bipyridine) bis 2-(2,5,8,11-tetraoxadodecanoyl)benzenesulfonate.
**Figure 2** shows the schematic synthesis of 4,4'-bis(2-(2-(2-methoxyethoxy)ethoxy)ethoxy)-6,6'-dimethyl-2,2'-bipyridine
**Figure 3** shows the molecular structure of a copper complex melt comprising [Cu(I)(tmbe)₂)](TFSI), where "tmbe" represents 4,4'-bis(2-(2-(2-methoxyethoxy)ethoxy)ethoxy)-6,6'-dimethyl-2,2'-bipyridine and the anion (TFSI⁻) is bis(trifluoromrthylsulfonyl)imide.
**Figure 4** shows UV-visible spectroscopy of copper complex melt [Cu(I)(tmbe)₂)](TFSI) dissolved in acetonitrile.

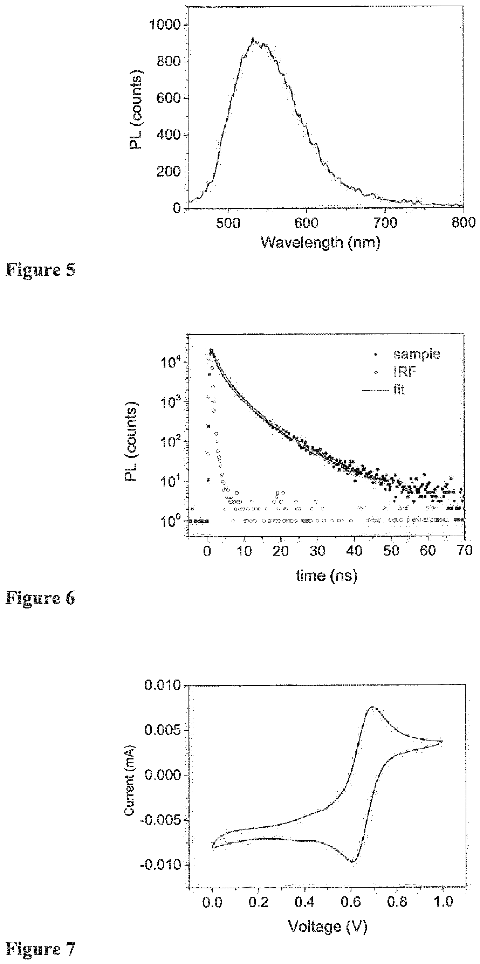

**Figure 5** shows photoluminescence (PL) of copper complex melt [Cu(I)(tmbe)₂)](TFSI) dissolved in acetonitrile.
**Figure 6** shows photoluminescence decay dynamics of copper complex melt [Cu(I)(tmbe)₂)](TFSI) dissolved in acetonitrile. The traces were collected at 540 nm with photoluminescence maximum following nanosecond laser pulsed excitation at 408 nm. The black open dot symbols are instrument response function. The gray line is bi-exponential fit of the data.
**Figure 7** shows cyclic voltammograms of 5 mM copper complex melt [Cu(I)(tmbe)₂)](TFSI) in 0.1 M LiTFSI acetonitrile solution. Potentials are referenced to the Ag/AgCl (saturated LiCl in ethanol) electrode.

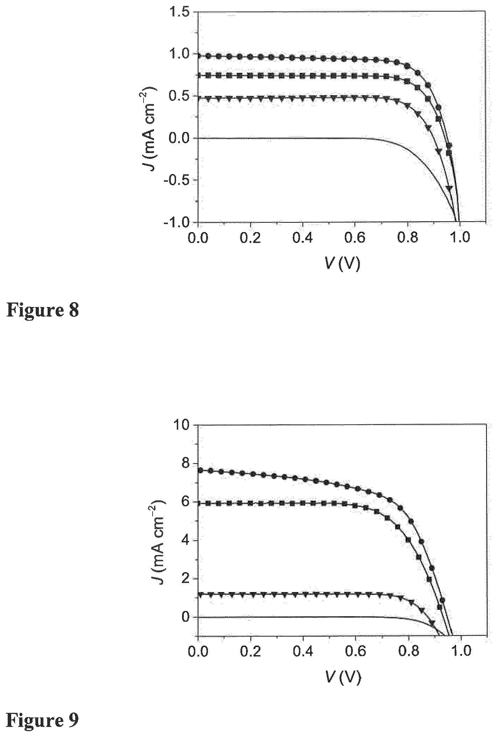

**Figure 8** shows *J-V* curves of a DSC based on copper complex melt [Cu(I)(tmbe)₂)](TFSI) hole transporter fabricated by evaporating solvents from Electrolyte I under dark (line) and standard AM1.5G with irradiation intensities of 1,000 (round), 500 (square) and 100 W/m² (triangle).
**Figure 9** shows *J-V* curves of a DSC based on copper complex melt [Cu(I)(4,4',6,6'-tetramethyl-2,2'-bipyridine)₂](2-(2,5,8,11-tetraoxadodecanoyl)benzamide sulfonate) hole transporter fabricated by evaporating solvents from Electrolyte II under dark (line) and standard AM1.5G with irradiation intensities of 1,000 (round), 500 (square) and 100 W/m² (triangle).

### Detailed Description

The present invention concerns a redox copper complex melt having a melting point below 100°C and comprising a metalorganic complex of formula (I)

[Cu (La)ₙ] (I),

wherein
n is an integer 2; and
La is a bidentate ligand selected from a ligand according to any one of formulae (1) and (2): 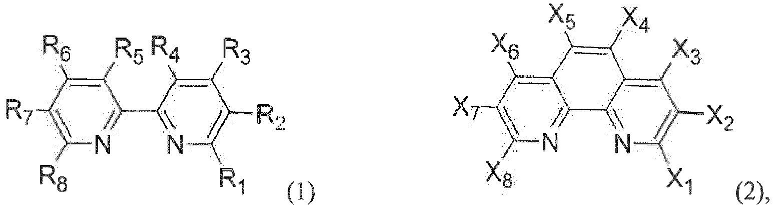 wherein:
   R₁, R₂, R₃, R₄, R₅, R₆, R₇, R₈, X₁, X₂, X₃, X₄, X₅, X₆, X₇, and X₈ are independently selected from H, halogen, Cl⁻, Br⁻, I⁻,-NO₂, -OH, -NH₂, -COOH, -CN, -OCN⁻, isocyanate group, sulfonyl group and from substituents comprising 1 to 40 carbons and 0 to 20 heteroatoms and/or groups selected from C1-C25 alkyl group, C4-C20 aryl group, C4-C20 arylalkyl group, C1-C25 alkoxy group, C4-C25 alkoxyalkoxyalkoxyalkyl group, C1-C20 heteroalkyl group, C4-C20 heteroaryl group, C4-C20 heteroarylalkyl group, said heteroatom being selected from N, S, or O, and from -O-, -O-R⁶, -O-R⁶-R⁶,-C(O)-O-R⁶, -C(O)-(NR⁷)-O-R⁶, -C(O)-(NR⁷)-R⁶; with R⁶ being independently selected from C1-C25 alkyl group or 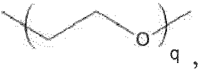 q being an integer from 1 to 20, and with R⁷ being selected from H or C1-C16 alkyl group, from-(CH₂)₂-O-, -C(O)-, -C(O)O-, -S-, -S(O)-, SO₂-, -S(O)₂O-, -N=, -P=, -NR⁹-,-PR⁹-, -P(O)(OR⁹)-, -P(O)(OR⁹)O-, -P(O)(NR⁹R⁹)-, -P(O)(NR⁹R⁹)O-,-P(O)(NR⁹R⁹)NR⁹-, -S(O)NR⁹-, and -S(O)₂NR⁹, with R⁹ being independently selected from H, C1-C6 alkyl, C4-C20 aryl group, C4-C20 arylalkyl group and C4-C20 heteroaryl group, wherein the heteroatom is selected from N, S, or O, and said alkyl, arylalkyl and heteroaryl groups being optionally perfluorinated.

R₁, R₂, R₃, R₄, R₅, R₆, R₇, and R₈ of of La ligand of formula (1) of the metalorganic complex of formula (I) and/or X₁, X₂, X₃, X₄, X₅, X₆, X₇, and X₈ of La ligand of formula (2) of the metalorganic complex of formula (I) are independently selected from H, halogen, Cl⁻, Br⁻, I⁻ ,-NO₂, -OH, -NH₂, -COOH, -CN, -OCN⁻, isocyanate group, sulfonyl group and substituents comprising 1 to 40 carbons and 0 to 20 heteroatoms and/or groups selected from C1-C6 alkyl group, C4-C20 aryl group, C4-C20 arylalkyl group, C1-C20 heteroalkyl group, C4-C20 heteroaryl group, C4-C20 heteroarylalkyl group, wherein the heteroatom is selected from N, S, or O, -O-, -((CH₂)₂-O-, -C(O)-, -C(O)O-, -S-, -S(O)-, SO₂-, -S(O)₂O-, -N=, -P=, - NR⁹-, -PR⁹-, -P(O)(OR⁹)-, -P(O)(OR⁹)O-, -P(O)(NR⁹R⁹)-, -P(O)(NR⁹R⁹)O- , - P(O)(NR⁹R⁹)NR⁹-, -S(O)NR⁹-, and -S(O)₂NR⁹, with R⁹ being independently selected from H, C1-C6 alkyl, C4-C20 aryl group, C4-C20 arylalkyl group and C4-C20 heteroaryl group, wherein the heteroatom is selected from N, S, or O, and said alkyl, arylalkyl and heteroaryl groups being optionally perfluorinated.

The redox copper complex melt comprises a metalorganic complex of the invention, wherein the copper metal may be in oxidation state (I) or in oxidation state (II). Said redox copper complex melt may comprise metalorganic complexes in both oxidation states (I) and (II) according to formula (I.1): [Cu(I/II) (La)ₙ] (I.1), wherein the copper metal is in oxidation state (I) and/or (II). The metalorganic complex of formula (I) may be under the form of a monovalent and/or divalent cation depending on the oxidation sate of the copper metal. Considering the oxidation states of the copper metal, the cationic form of the metalorganic complex of formula (I) may be represented by formula (1.2), [Cu (La)ₙ]^{+/++} (I.2).

The redox copper complex melt comprises a metalorganic complex according to anyone of formulae (Ia) and (Ib) 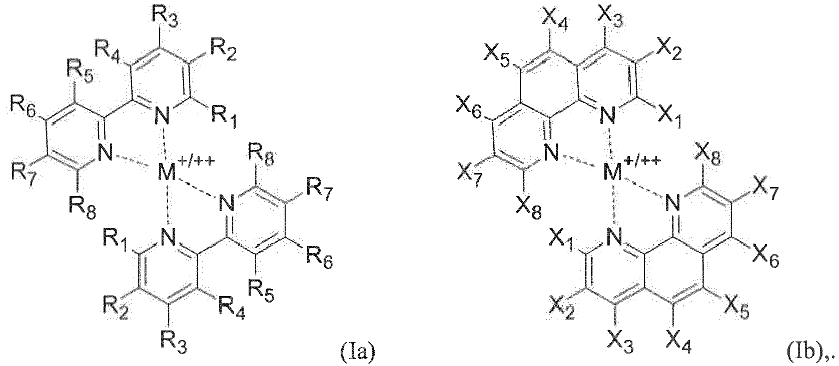 wherein metal M is copper (Cu). Said Cu metal is under the oxidation sate (I) or (II), namely Cu (I/II) or Cu(I)/Cu(II) or Cu⁺/Cu⁺⁺ or Cu^{+/++}.

Some non-linear molecular systems in a degenerate electronic state will be unstable and will undergo distortion to form a system of lower symmetry and lower energy and become stable. The metalorganic complex of formula (I) may have a distorted tetragonal geometry according to any one of the formulae (Ic) and (Id) below

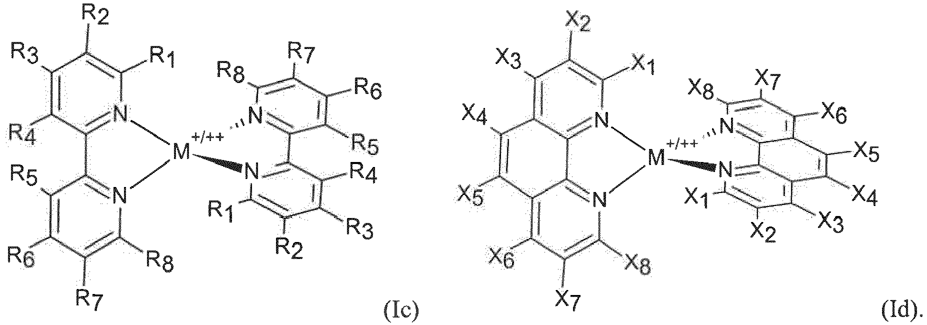

In one embodiment, R₂, R₄, R₅ and R₇ of La ligand of formula (1) of the metalorganic complex of formula (I) and/or X₂, X₄, X₅, and X₇ of La ligand of formula (2) of the metalorganic complex of formula (I) are H.

In a further embodiment, R₁ and R₈ of La ligand of formula (1) of the metalorganic complex of formula (I) and/or X₁ and X₈ of La ligand of formula (2) of the metalorganic complex of formula (I) are selected from H or substituents comprising 1 to 40 carbons and 0 to 20 groups selected from C1-C25 alkyl group, C4-C20 aryl group, C4-C20 arylalkyl group. R₁ and R₈ of La ligand of formula (1) and/or X₁ and X₈ of La ligand of formula (2) may be selected from H, C1-C25 alkyl group, C4-C20 aryl group, C4-C20 arylalkyl group, C1-C20 heteroalkyl group, C4-C20 heteroaryl group, C4-C20 heteroarylalkyl group, wherein the heteroatom is selected from N, S, or O. R₁ and R₈ of La ligand of formula (1) and/or X₁ and X₈ of La ligand of formula (2) may be further selected from C1-C6 alkyl group, C4-C20 aryl group, C4-C20 arylalkyl group. R₁ and R₈ of La ligand of formula (1) and/or X₁ and X₈ of La ligand of formula (2) are selected from C1-C25 alkyl group, C1-C25 alkoxy group, C2-C25 alkoxyalkyl group, and methyl group or comprise 1 to 4 groups selected from C1-C25 alkyl group, C1-C25 alkoxy group, C2-C25 alkoxyalkyl group, and methyl group. R₁ and R₈ of La ligand of formula (1) and/or X₁ and X₈ of La ligand of formula (2) are methyl groups.

According to another embodiment, R₃ and R₆ of La ligand of formula (1) and/or X₃ and X₆ of La ligand of formula (2) are selected from H or from substituents comprising 1 to 40 carbons and 1 to 20 heteroatoms and/or groups selected from C1-C25 alkyl group, C4-C20 aryl group, C4-C20 arylalkyl group, C1-C25 alkoxy group, C4-C25 alkoxyalkoxyalkoxyalkyl group, C1-C20 heteroalkyl group, C4-C20 heteroaryl group, C4-C20 heteroarylalkyl group, said heteroatom being selected from N, S, or O, and from -O-, - O-R⁶, -O-R⁶-R⁶,-C(O)-O-R⁶, -C(O)-(NR⁷)-O-R⁶, -C(O)-(NR⁷)-R⁶; with R⁶ being independently selected from C1-C25 alkyl group or 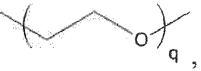 q being an integer from 1 to 20, and with R⁷ being selected from H or C1-C16 alkyl group, from -(CH₂)₂-O-, - C(O)-, -C(O)O-, and -NR⁹-, with R⁹ being independently selected from H, C1-C6 alkyl, C4-C20 aryl group, C4-C20 arylalkyl group and C4-C20 heteroaryl group, wherein the heteroatom is selected from N, S, or O.

In a further embodiment, R₃ and R₆ of La ligand of formula (1) and/or X₃ and X₆ of La ligand of formula (2) are selected from substituents comprising 1 to 40 carbons and 1 to 20 heteroatoms and/or groups selected from C1-C25 alkoxy group, C4-C25 alkoxyalkoxyalkoxyalkyl group, C1-C20 heteroalkyl group, C4-C20 heteroaryl group, C4-C20 heteroarylalkyl group, said heteroatom being selected from N, S, or O, and from -O-,-O-R⁶, -O-R⁶-R⁶,-C(O)-O-R⁶, -C(O)-(NR⁷)-O-R⁶, -C(O)-(NR⁷)-R⁶; with R⁶ being independently selected from C1-C25 alkyl group or 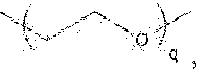 q being an integer from 1 to 20, and with R7 being selected from H or C1-C16 alkyl group, from -(CH₂)₂-O-, - C(O)-, -C(O)O-, and -NR⁹-, with R⁹ being independently selected from H, C1-C6 alkyl, C4-C20 aryl group, C4-C20 arylalkyl group and C4-C20 heteroaryl group, wherein the heteroatom is selected from N, S, or O.

"q" may be an integer from 1 to 15, 1 to 10, 1 to 5, 4 to 18, or from 1, 2, 3 or 4. When R⁶ is present twice or more in the substituting moiety, each said R⁶ may be identical or different, preferably different.

R₃ and R₆ of La ligand of formula (1) and/or X₃ and X₆ of La ligand of formula (2) may be further selected from substituents comprising 1 to 40 carbons and 1 to 20 heteroatoms and/or groups selected from C1-C6 alkyl group, C4-C20 aryl group, C4-C20 arylalkyl group, C1-C20 heteroalkyl group, C4-C20 heteroaryl group, C4-C20 heteroarylalkyl group, wherein the heteroatom is selected from N, S, or O, -O-, -(CH₂)₂-O-, -C(O)-, -C(O)O-, and -NR⁹-, with R⁹ being independently selected from H, C1-C6 alkyl, C4-C20 aryl group, C4-C20 arylalkyl group and C4-C20 heteroaryl group, wherein the heteroatom is selected from N, S, or O.

R₃ and R₆ of La ligand of formula (1) and/or X₃ and X₆ of La ligand of formula (2) are selected from C1-C20 heteroalkyl group, C4-C20 heteroaryl group, C4-C20 heteroarylalkyl group, said groups comprising 1-10 or 1-6 heteroatom, wherein the heteroatom is selected from N, S, or O, or selected from substituents comprising 4 to 20 carbons and 1 to 10 or 1 to 6 groups selected from -O-, -(CH₂)₂-O-, -C(O)-, -C(O)O-, and -NR⁹-, with R⁹ being independently selected from H or C1-C6 alkyl. R₃ and R₆ of La ligand of formula (1) and/or X₃, and X₆ of La ligand of formula (2) are further selected from C1-C25 alkoxy group, C4-C25 alkoxyalkoxyalkoxyalkyl group, -O-R⁶, -O-R⁶-R⁶,-C(O)-O-R⁶, -C(O)-(NR⁷)-O-R⁶, - C(O)-(NR⁷)-R⁶; with R⁶ being independently selected from C1-C25 alkyl group or 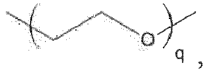 q being an integer from 1 to 20, and with R⁷ being selected from H or C1-C16 alkyl group. R₃ and R₆ of La ligand of formula (1) and/or X₃, and X₆ of La ligand of formula (2) are further selected from C1-C25 alkoxy group, C4-C25 alkoxyalkoxyalkoxyalkyl group, -O-R⁶, -O-R⁶-R⁶,-C(O)-O-R⁶, -C(O)-(NR⁷)-O-R⁶, -C(O)-(NR⁷)-R⁶; with R⁶ being indepently selected from C1-C25 alkyl group or 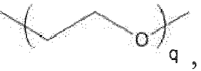 q being an integer from 1 to 20, and with R⁷ being selected from H or C1-C16 alkyl group. R₃ and R₆ of La ligand of formula (1) and/or X₃ and X₆ of La ligand of formula (2) are further selected from substituents comprising 1 to 20 groups selected from C1-C25 alkoxy group, C4-C25 alkoxyalkoxyalkoyalkyl group, C3-C25 alkoxyalkoxyalkyl group, methoxyethanyl group, 1-methoxy-2-[2-(2-methoxyethoxy)ethoxy]ethanyl group, 1-methoxy-2-(2-methoxyethoxy)ethanyl group, 1,2-dimethoxyethanyl group or 2-(2-(2-methoxyethoxy)ethoxy)ethanyl group.

R₃ and R₆ of La ligand of formula (1) and/or X₃ and X₆ of La ligand of formula (2) may be further selected from H or substituents comprising 1 to 40 carbons and 0 to 20 groups selected from C1-C25 alkyl group, C1-C25 alkoxy group, C2-C25 alkoxyalkyl group, C4-C20 aryl group, C4-C20 arylalkyl group, C4-C25 alkoxyaryl group, C5-C25 alkoxyarylalkyl group, C6-C25 alkoxyarylalkoxyalkyl group. R₃ and R₆ of La ligand of formula (1) and/or X₃ and X₆ of La ligand of formula (2) may be further selected from C1-C25 alkyl group, C1-C25 alkoxy group, C2-C25 alkoxyalkyl group, and methyl group. The substituents of R₃ and R₆ of La ligand of formula (1) and/or the substituents of X₃ and X₆ of La ligand of formula (2) may be identical to the substituents of R₁ and R₈ of La ligand of formula (1) and/or the substituents of X₁ and X₈ of La ligand of formula (2), respectively.

According to another embodiment, La ligand of formula (1) is 4,4'-bis(2-(2-(2-methoxyethoxy)ethoxy)ethoxy)-6,6'-dimethyl-2,2'-bipyridine.

According to a further embodiment, La ligand of formula (2) is 4,7-bis(2-(2-(2-methoxyethoxy)ethoxy)ethoxy)-2,9-dimethyl-1,10-phenanthroline.

In a further embodiment, the metalorganic complex of formula (I) is cationic and the redox metal complex melt further comprises a negative counterion selected from ClO₄⁻, PF₆⁻, BF₄⁻, [B(CN)₄]⁻, CF3SO₃⁻, [(CF₃SO₂)₂N]⁻ (TFSI), [B(C₆H₃(m-CF₃)₂)₄]⁻, [B(C₆F₅)₄]⁻, [B(C₆H₅)₄]⁻, [Al(OC(CF₃)₃)₄]⁻, [CB₁₁H₁₂]⁻, and a moiety according to formula (3) 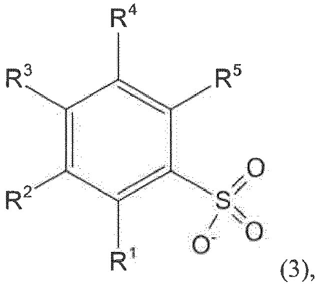 wherein R¹, R², R³, R⁴, R⁵ are independently selected from H, -CF₃ and substituents comprising 1 to 40 carbons and 0 to 20 groups selected from C1-C16 alkyl group, C1-C25 alkoxy group, C4-C25 alkoxyalkoxyalkoxyalkyl group, C4-C20 aryl group, C4-C20 arylalkyl group, -O-, -CF₃, -O-R⁶, -O-R⁶-R⁶,-C(O)-O-R⁶, -C(O)-(NR⁷)-O-R⁶, -C(O)-(NR⁷)-R⁶, with R⁶ being independently selected from C1-C25 alkyl group or 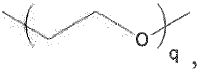 q being an integer from 1 to 20, and with R⁷ being selected from H or C1-C16 alkyl group; or from a salt of a moiety of formula (3).

The moiety according to formula (3) may be further selected from a moiety according to anyone of formulae (4) to (8) 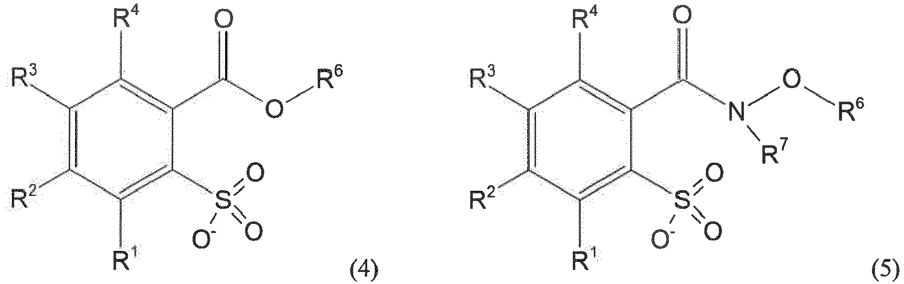 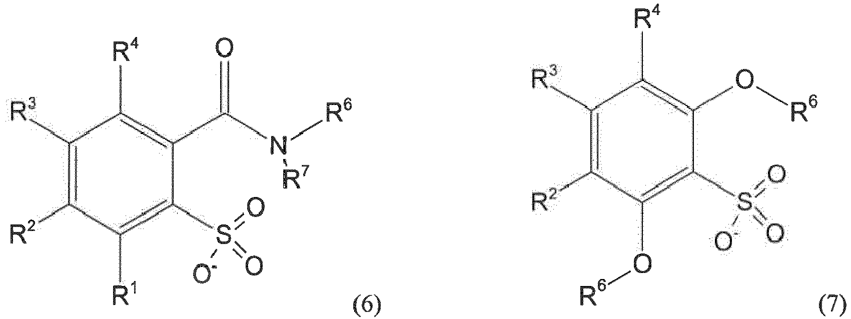 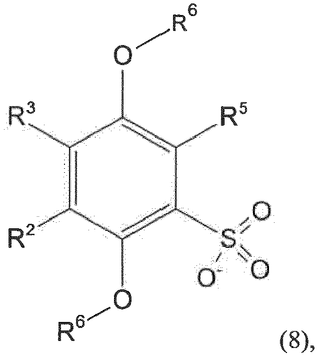 wherein R⁶ and R⁷, if present, are defined as above and R¹, R², R³, R⁴, and R⁵, if present, are defined as above. Namely, R¹, R², R³, R⁴, R⁵ are independently selected from H, -CF₃ and substituents comprising 1 to 40 carbons and 0 to 20 groups selected from C1-C16 alkyl group, C1-C25 alkoxy group, C4-C25 alkoxyalkoxyalkoxyalkyl group, C4-C20 aryl group, C4-C20 arylalkyl group, -O-, -CF₃, -O-R⁶, -O-R⁶-R⁶,-C(O)-O-R⁶, -C(O)-(NR⁷)-OR⁶, -C(O)-(NR⁷)-R⁶, with R⁷ being independently selected from C1-C25 alkyl group or 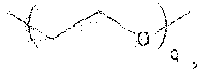 q being an integer from 1 to 20, and with R⁷ being selected from H or C1-C16 alkyl group. In particular R¹, R², R³, and R⁴ of a moiety according to any one of formulae (4) to (6), R² and R⁴ of moiety of formula (7) and R² and R⁵ of moiety of formula (8) may be H; R³ of moiety of formula (7) or moiety of formula (8) may be selected from H or -CF₃, R⁶ of moieties of any one formulae (4) to (8) being independently selected from C1-C25 alkyl group or 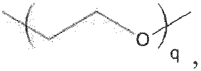 q being an integer from 1 to 20, and R⁷ of moieties of any one of formulae (5) and (6) being selected from H or C1-C16 alkyl group.

According to a further embodiment, the negative counter ion in the redox metal complex melt is selected from [(CF₃SO₂)₂N]⁻ (TFSI) or a moiety according to any one of formulae (3), and (4) to (8). The negative counter ion is selected from a moiety according to any one of formulae (3), and (4) to (8).

Without to be bound by theory, it is believed that the substitution of La ligand according to anyone of formulae (1) or (2) with carbon chain (linear or branched) comprising alkoxy groups or ether groups or heteroatom such as oxygen or a polymer of methyl ethyl ether changes and decreases the melting point of the metalorganic complex. In addition to the substitution of La ligand or as an alternative to obtain a melt of a metalorganic complex of formula (I), the negative counterion of the complex being selected from a moiety according to any one of formulae (3), and (4) to (8), forming therefore the redox metal complex, also changes and decreases the melting point of the metalorganic complex and leads to provide a melt of such a complex and therefore a redox copper complex melt. The use of steps of evaporation, in particular by rotavap, in the synthesis process of the redox metal complex melt to concentrate and evaporate solvents or liquids also facilitates obtaining an oil or melt comprising the metalorganic complex.

According to one embodiment, the negative counter ion of the metalorganic complex is provided from a salt of moiety (3), wherein said salt comprises a cation selected from Na⁺, Li⁺, Cs⁺ and K⁺.

The number of anion or negative counterion to obtain an uncharged/neutral metalorganic complex depends on the oxidation state of the copper metal ion and thus valence of the cationic metalorganic complex.

The invention also provides an optoelectronic and/or electrochemical device or redox flow batteries comprising at least one or more redox copper complex melt according to the invention.

The optoelectronic and/or electrochemical device of the invention is selected from an organic photovoltaic device, a photovoltaic solid state device, a p-n heterojunction, an organic solar cell, a dye sensitized solar cell, a phototransistor and OLED (organic light-emitting diode). The optoelectronic and/or electrochemical device is selected from a photoelectric conversion device, a photovoltaic cell or a dye-sensitized solar cell.

According to a further embodiment, the optoelectronic and/or electrochemical device of the invention comprises an organic charge transporting layer, said organic charge transporting layer comprising an organic charge transport material and at least one complex of the invention, namely a redox metal complex melt as defined above. Said complex melt of the invention may be used as dopant in the organic charge transport material and forms with the organic charge transport material, which may be selected from organic electron and/or hole transporting material, a doped organic charge transport material.

According to one embodiment, the redox copper complex melt is used as one component of electrolyte or hole transporting material in the optoelectronic and/or electrochemical device of the invention.

The electrolyte of the optoelectronic and/or electrochemical device or redox flow batteries comprises one or more redox copper complex melts. The electrolyte or the redox melt electrolyte may comprise a Cu(I) to Cu(II) concentration expressed in a Cu(I):Cu(II) or Cu(I) to Cu(II) ratio (or molar ratio in percentage) being selected in the range from 100:0 to 30:70, from 99:1 to 40:60, from 90:10 to 50:50, from 80:20 to 60:40, from 70:30 to 20:80, from 40:60 to 10:90, or from 20:80 to 1:99. It may be that the more the concentration of Cu(I) in the redox copper complex melt increases, the more the performance of the device increases and the more the concentration of Cu(II) increases, the more the stability of the device increases. The concentration of Cu(I) and Cu(II) in the electrolyte depends on a compromise between performance and stability of the device.

The optoelectronic and/or electrochemical device may further comprise a conducting support layer, n-type semiconductor, a light-absorber layer or a sensitizer layer and a counter electrode and/or metal layer. The optoelectronic and/or electrochemical device may further comprise a hole transporting material. The optoelectronic and/or electrochemical device may comprise an optional surface-increasing scaffold structure. Said metal layer may be doped as well as the n-type and/or the p-type semiconductor. A conductive layer comprising a conductive material may be present between the hole transporting layer and the counter electrode and/or metal layer. The hole transporting layer may be provided on the sensitizer layer and is between the sensitizer layer and the conducting current providing layer, if present, or the counter electrode and/or metal layer. Further layer may be present.

The conductive material is selected from one or more conductive polymers or one or more hole transporting materials, which may be selected from poly(3,4-ethylenedioxythiophene):poly(styrenesulfonate) (PEDOT:PSS), poly(3,4-ethylenedioxythiophene):poly(styrenesulfonate):grapheme nanocomposite (PEDOT:PSS:graphene), poly(N-vinylcarbazole) (PVK) and sulfonated poly(diphenylamine) (SPDPA), preferably from PEDOT:PSS, PEDOT:PSS:graphene and PVK, more preferably from PEDOT:PSS. Conductive polymers may also be selected from polymers comprising polyaniline, polypyrrole, polythiophene, polybenzene, polyethylenedioxythiophene, polypropylenedioxy-thiophene, polyacetylene, and combinations of two or more of the aforementioned, for example.

The conducting support layer is preferably substantially transparent. "Transparent" means transparent to at least a part, preferably a major part of the visible light. Preferably, the conducting support layer is substantially transparent to all wavelengths or types of visible light. Furthermore, the conducting support layer may be transparent to non-visible light, such as UV and IR radiation, for example.

According to an embodiment, the conducting support layer provides the support layer of photovoltaic solid state device. Preferably, the optoelectronic and/or electrochemical device is built on said support layer. The support of the device may be also provided on the side of the counter electrode. In this case, the conductive support layer does not necessarily provide the support of the device, but may simply be or comprise a current collector, for example a metal foil.

The conducting support layer preferably functions and/or comprises a current collector, collecting the current obtained from the device. The conducting support layer may comprise a material selected from indium doped tin oxide (ITO), fluorine doped tinoxide (FTO), ZnO-Ga₂O₃, ZnO-Al₂O₃, tin-oxide, antimony doped tin oxide (ATO), SrGeO₃ and zinc oxide, preferably coated on a transparent substrate, such as plastic or glass. In this case, the plastic or glass provides the support structure of the layer and the cited conducting material provides the conductivity. Such support layers are generally known as conductive glass and conductive plastic, respectively, which are thus preferred conducting support layers in accordance with the invention. The conducting support layer comprises a conducting transparent layer, which may be selected from conducting glass and from conducting plastic.

The surface-increasing scaffold structure is provided on said conducting support structure or on a protective layer that may be provided on said scaffold structure. The surface-increasing scaffold structure is nanostructured and/or mesoporous. The scaffold structure is made from and/or comprises a metal oxide. For example, the material of the scaffold structure is selected from semiconducting materials, such as Si, TiO₂, SnO₂, ZrO₂, Al₂O₃, Fe₂O₃, ZnO, WO₃, Nb₂O₅, CdS, ZnS, PbS, Bi₂S₃, CdSe, CdTe, SrTiO₃, GaP, InP, GaAs, CuInS₂, CuInSe₂, and combinations thereof, for example. Preferred semiconductor materials are Si, TiO₂, SnO₂, ZnO, WO₃, Nb₂O₅ and SrTiO₃, for example. According to an embodiment, the surface-increasing scaffold structure is nanostructured and/or nanoporous.

The invention does not intend to exclude the possibility that there are one or more intermediate layers between the scaffold structure and the conductive support. Such intermediate layers, if present, would preferably be conducting and/or semiconducting.

According to an embodiment, the sensitizer layer of the photoelectrochemical and/or optoelectronic device comprising at least one pigment being selecting from organic, inorganic, organometallic and organic-inorganic pigments or a combination thereof. The sensitizer is preferably a light absorbing compound or material. Preferably, the sensitizer is a pigment, and most preferably the sensitizer is an organic-inorganic pigment.

The sensitizer may, for example, comprise at least one dye disclosed in WO2004/097871A1 or suitable ruthenium dyes are disclosed, for example, in WO2006/010290. The sensitizer may either be a light absorber comprising at least one perovskite material as disclosed in WO 2014/020499A1, WO 2015/087210A1 and/or WO 2016/055025A1.

The sensitizer layer or light-harvester layer may comprise one or more pigments of the group consisting of organometallic sensitizing compounds (phthalocyanine derived compounds, phtalocyanine compounds as disclosed in WO2008/145172A1, porphyrin derived compounds, porphyrin compounds as disclosed in WO2013057538A1), metal free organic sensitizing compounds (diketopyrrolopyrrole (DPP) based sensitizer, sensitizers as recited in WO 2014/033582 A2 and in EP2511924), inorganic sensitizing compounds such as quantum dots, Sb₂S₃ (Antimonysulfide, for example in the form of thin films), aggregates of organic pigments, nanocomposites, in particular organic-inorganic perovskites, and combinations of the aforementioned.

The term "perovskite", for the purpose of this specification, refers to the "perovskite structure" and not specifically to the perovskite material, CaTi03. For the purpose of this specification, "perovskite" encompasses and preferably relates to any material that has the same type of crystal structure as calcium titanium oxide and of materials in which the bivalent cation is replaced by two separate monovalent cations. The perovskite structure has the general stoichiometry AMX₃, where "A" and "M" are cations and "X" is an anion. The "A" and "M" cations can have a variety of charges and in the original Perovskite mineral (CaTiO₃), the A cation is divalent and the M cation is tetravalent. For the purpose of this invention, the perovskite formulae includes structures having three (3) or four (4) anions, which may be the same or different, and/or one or two (2) organic cations, and/or metal atoms carrying two or three positive charges, in accordance with the formulae presented elsewhere in this specification.

According to an embodiment, the photovoltaic device of the invention comprises one or more layer of an organic-inorganic perovskite. Said organic-inorganic perovskite may be selected from organic-inorganic perovskite described in WO 2014/020499A1, WO 2015/001459A1, WO 2015/107454A1, WO 2015/114521A1, PCT/IB2017/051538, EP 16180656.7.

The counter electrode and/or metal layer and/or back contact generally comprises a catalytically active material, suitable to provide electrons and/or fill holes towards the inside of the device. The back contact may comprise one or more materials selected from Pt, Au, Ni, Cu, Ag, In, Ru, Pd, Rh, Ir, Os, porous carbon (C), conductive polymer and a combination of two or more of the aforementioned. Conductive polymers may be selected from polymers comprising poly(3,4-ethylenedioxythiophene):poly(styrenesulfonate) (PEDOT:PSS), polyaniline, polypyrrole, polythiophene, polybenzene, polyethylenedioxythiophene, polypropylenedioxy-thiophene, polyacetylene, and combinations of two or more of the aforementioned, for example.

The present invention also provides a method for producing a redox copper complex melt comprising a metal organic complex of formula (I):
- providing a solution comprising ligand La selected from formula (1) or from formula (2), and further comprising a salt of copper;
- exchanging the anion provided by the copper salt by titration with a solution comprising an anion ClO₄⁻, PF₆⁻, BF₄⁻, [B(CN)₄]⁻, CF3SO₃⁻, [(CF₃SO₂)₂N]⁻ (TFSI), [B(C₆H₃(m-CF₃)₂)₄]⁻, [B(C₆F₅)₄]⁻, [B(C₆H₅)₄]⁻, [Al(OC(CF₃)₃)₄]⁻, [CB₁₁H₁₂]⁻, a moiety according to formula (3) 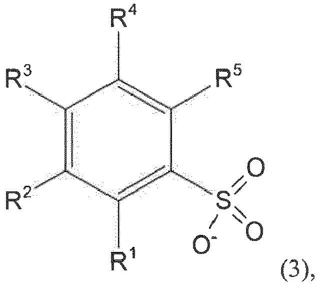 wherein R¹, R², R³, R⁴, R⁵ are independently selected from H, -CF₃ and substituents comprising 1 to 40 carbons and 0 to 20 groups selected from C1-C16 alkyl group, C1-C25 alkoxy group, C4-C25 alkoxyalkoxyalkoxyalkyl group, C4-C20 aryl group, C4-C20 arylalkyl group, -O-, -CF₃, -O-R⁶, -O-R⁶-R⁶,-C(O)-O-R⁶, -C(O)-(NR⁷)-OR⁶, -C(O)-(NR⁷)-R⁶, with R⁶ being independently selected from C1-C25 alkyl group or 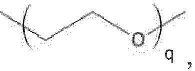 q being an integer from 1 to 20, and with R⁷ being selected from H or C1-C16 alkyl group; or a salt of a moiety of formula (3);
and- evaporating the titrated crude solution to obtain a melt comprising the redox copper complex melt.

The method of the invention further comprises a step of purifying said oil by chromatography followed by providing evaporation of obtained crude filtrate; and a step of filtrating the evaporated crude filtrate followed by providing evaporation of the last filtrate to obtain pure redox metal complex melt. The evaporation steps are performed by rotavaporator.

The present invention will now be illustrated by way of examples. These examples do not limit the scope of this invention, which is defined by the appended claims.

### Examples:

### Example 1:

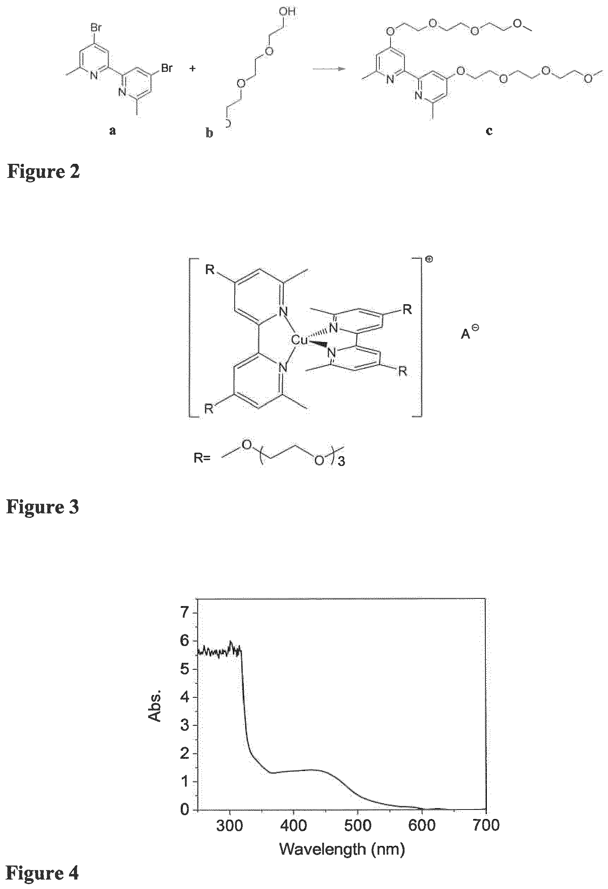

### Synthesis of 4,4'-bis(2-(2-(2-methoxyethoxy)ethoxy)ethoxy)-6,6'-dimethyl-2,2'-bipyridine (see Figure 2)

Sodium hydride (0.170 g, 7.02 mmol, 3 eq.) was added to 20 mL of anhydrous DMF at 0°C. The resulting suspension was stirred for 10 minutes after which 2-(2-(2-methoxyethoxy)ethoxy)ethan-1-ol (1.54 g, 9.36 mmol, 4 eq.) (compound b, Figure 2) was added drop wise to afford the formation of hydrogen gas. The solution was then left at 0°C for 2 hours to allow complete evolution of hydrogen gas. After that, 4,4'-dibromo-6,6'-dimethyl-2,2'-bipyridine (0.7 g, 2.05 mmol, 1 eq.) was added to the opaque mixture and the ice bath was removed to allow the reaction mixture to slowly warm up to room temperature. The beige opaque suspension was then heated at 90°C overnight. The resulting dark green mixture was then cooled down to RT, quenched with saturated a solution of ammonium chloride (200 ml) and the organics were extracted with DCM (3x100 mL). DCM was then removed and the residue dissolved in diethyl ether (100 mL), washed with deionized water (3x100 mL) and a saturated solution of sodium chloride (100 mL) to fully remove the high boiling point solvent DMF. The obtained yellow solution was dried over magnesium sulfate followed by evaporation of the diethyl ether. The product was obtained as a greenish wax 0.970 g (81 % yield).

¹H NMR (400 MHz, Chloroform-*d*) δ 7.79 (s, 2H), 6.72 (s, 2H), 4.29 (t, *J* = 4.8 Hz, 4H), 3.91 (t, *J* = 4.8 Hz, 4H), 3.81 - 3.64 (m, 12H), 3.57 (t, *J* = 4.7 Hz, 4H), 3.40 (s, 6H), 2.57 (s, 6H); ¹³C NMR (101 MHz, Chloroform-*d*) δ 166.04, 159.22, 157.52, 110.04, 104.49, 71.95, 70.91, 70.69, 70.61, 69.45, 67.24, 59.05, 24.73; MS (APPI⁺, *m*/*z*): [M+H⁺] calculated: 509.2857 found: 509.2849

### Synthesis of sodium 2-(2,5,8,11-tetraoxadodecanoyl)benzenesulfonate, a salt of a counterion ion of formula (4)

3H-benzo[c][1,2]oxathiol-3-one 1,1-dioxide (4.846 g, 26.31 mmol, 1 eq.) and 2-(2-(2-methoxyethoxy)ethoxy)ethan-1-ol (4.320 g, 26.31 mmol, 1 eq.) (see Figure 2) was charged in a flask with dichloromethane (20 mL) and stirred at room temperature for 24 hours. Then, An additional 0.5 g of 3H-benzo[c][1,2]oxathiol-3-one 1,1-dioxide was added and the suspension was stirred at room temperature for 2 extra days. The excess of solid was removed by filtration and dicholormethane evaporated under reduced pressure. The obtained pink thick oil was neutralized with Na₂CO₃ 5% aq. until no formation of CO₂. Then, water was removed under reduced pressure. Sonication of the obtained slurry in EtOH afforded the precipitation of Na₂CO₃ which was removed by filtration. The latter procedure was repeated 3 times and the desired product was obtained as a beige waxy solid. 8.08 g (83%)

¹H NMR of the intermediate (400 MHz, Acetonitrile-*d*₃) δ 8.09 (d, *J* = 7.1 Hz, 1H), 7.80 - 7.70 (m, 2H), 4.49 (dd, *J* = 5.6, 3.6 Hz, 2H), 3.97 (dd, *J* = 5.6, 3.5 Hz, 2H), 3.81 (dd, *J* = 5.6, 3.5 Hz, 2H), 3.72 - 3.64 (m, 2H), 3.59 (s, 3H), 3.58 - 3.45 (m, 3H), 3.33 (s, 3H). Mass Spectrometry (ESI⁻, *m*/*z*): [M-H⁺] calculated: 347.0795 found: 342.0797

¹H NMR of the product (400 MHz, Acetonitrile-*d*₃) δ 7.94 (d, *J* = 7.6 Hz, 1 H), 7.72 - 7.29 (m, 3H), 4.52 - 4.37 (m, 2H), 3.86 - 3.79 (m, 2H), 3.72 - 3.46 (m, 8H), 3.35 (d, *J* = 1.6 Hz, 3H).Mass Spectrometry (ESI⁺, m/z): [M+Na⁺] calculated: 393.0591 found: 393.0580

### Synthesis of a Copper (I) redox complex melt: [Cu(I)(4,4'-bis(2-(2-(2-methoxyethoxy)ethoxy)ethoxy)-6,6'-dimethyl-2,2'-bipyridine)₂)] (TFSI)

To a 25 mL ethanol solution of CuCl (24 mg; 0.23 mmol) was added [tmbe] (950 mg; 0.69 mmol). The brown slurry was stirred under nitrogen for 24h. A brown solution formed which slowly became red. The chloride counterion was replaced by bis(trifluoromethane)sulfonimide (TFSI) titration using LiTFSI in a 12eq excess in acetonitrile. Flash evaporation of the crude led to an oil, which was chromatographed on a short silica column, eluting with DMF. A main red band was separated from a briskly eluting green impurity and a sluggishly eluting orange-red impurity. Flash evaporation gave an oil, which was redissolved in acetonitrile (10 mL), and the solution was filtered to remove silica. The filtrate was flash-evaporated and dried at 70 °C in vacuum for several days, yielding a red wax.

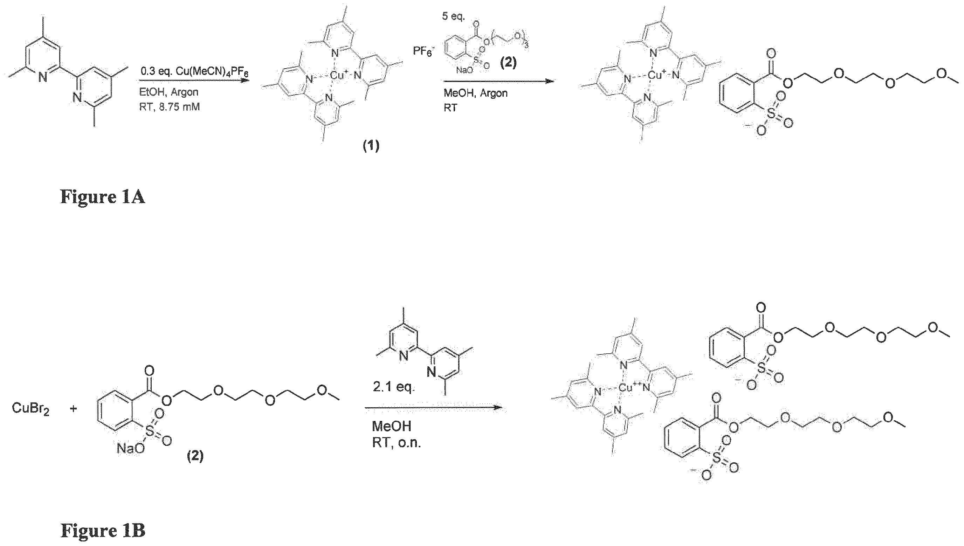

### Synthesis of Copper(I) bis(4,4',6,6'-tetramethyl-2,2'-bipyridine) 2-(2,5,8,11-tetraoxadodecanoyl)benzenesulfonate (see Figure 1A)

Copper(I)tetrakis acetonitrile hexafluorophosphate (0.3 eq., 0.8 mmol, 300 mg) was added to 20 mL of Argon degassed Ethanol. Then, 4,4',6,6'-tetramethyl-2,2'-bipyridine (1 eq., 2.41 mmol, 513 mg) was added to the solution which turned instantaneously red. Overnight stirring at room temperature afforded the formation of a red solid that was collected by filtration and dried under vacuum. The intermediate, Copper(I) bis(4,4',6,6'-tetramethyl-2,2'-bipyridine) hexafluorophosphate (compound (1)) was collected as a red crystalline solid 429 mg (84 %).

¹H NMR of the intermediate (1) (400 MHz, Acetone-*d*₆) δ 8.37 (s, 4H), 7.47 (s, 4H), 2.54 (s, 12H), 2.27 (s, 12H). ¹H NMR (400 MHz, Acetonitrile-*d*₃) δ 8.13 (s, 4H), 7.34 (s, 4H), 2.50 (s, 12H), 2.19 (s, 13H)(overlap with H₂O peak at 2.17 ppm). ¹³C NMR (101 MHz, Acetonitrile-*d*₃) δ 156.89, 151.75, 150.04, 126.18, 120.13, 23.98, 20.32. Mass Spectrometry (ESI⁺, *m*/*z*): [M-PF6⁻]⁺ calculated: 487.1917 found: 487.1923

Copper(I) bis(4,4',6,6'-tetramethyl-2,2'-bipyridine) hexafluorophosphate (compound (1)) (1 eq., 63 mmol, 400 mg) was solubilized in dry methanol followed by the addition of Sodium 2-(2,5,8,11-tetraoxadodecanoyl)benzenesulfonate (compound (2)) (5 eq., 3.16 mmol, 1.17 g) and stirred at RT under Argon for one day. Then, the solvent was removed under reduced pressure and dichloromethane was added to afford the precipitation of a solid. The solid was removed from the red solution by filtration. Evaporation of dichloromethane followed by several washing of the crude with dry acetone to remove the excess of anion, afforded the desired product as a very thick and viscous oil 405 mg (76%).
Mass Spectrometry (ESI⁺, *m*/*z*): [M-**(2)**⁻]⁺ calculated: 487.1917 found: 487.1792
Mass Spectrometry (ESI⁻, *m*/*z):* [**(2)**]- calculated: 347.0806 found: 347.0819

### Synthesis of Copper(II) bis (4,4',6,6'-tetramethyl-2,2'-bipyridine) bis 2-(2,5,8,11-tetraoxadodecanoyl)benzenesulfonate (see Figure 1B)

Copper(II) bromide (1.0 eq., 0.22 mmol, 50 mg) was added to 5 mL of Acetonitrile along with Sodium 2-(2,5,8,11-tetraoxadodecanoyl)benzenesulfonate **(2)** (2.1 eq., 0.47 mmol, 0.162 g). The resulting green solution was stirred for 6 hours followed by evaporation of the solvent under reduced pressure. Dichloromethane was added to trigger precipitation of a solid which was subsequently removed by filtration of the green solution. The solvent was removed by evaporation under reduced pressure and the green thick oil obtained was immediately dissolved in 10 mL of Methanol. Then, 4,4',6,6'-tetramethyl-2,2'-bipyridine (2.1 eq., 0.41 mmol, 99 mg) was added to the solution which intensified the green color. After stirring for 2 hours at room temperature, Methanol was removed and the crude washed with diethyl ether to remove the excess of 4,4',6,6'-tetramethyl-2,2'-bipyridine. The volatiles were removed by evaporation under reduced pressure and the desired product was obtained as a thick waxy dark green solid 218 mg (82%)
Mass Spectrometry (ESI⁺, *m*/*z*): [M-2×**(2)**⁻+e⁻]⁺ calculated: 487.1917 found: 487.2017
Mass Spectrometry (ESI⁻, *m*/*z*): [**(2)**]- calculated: 393.0591 found: 393.0580

### Example 2:

### Fabrication of solar cells

The 6.5 µm-thick mesoporous TiO₂ films (3.5 µm transparent layer + 4.0 µm light scattering layer) were immersed in 100 µM Y123 solution in acetonitril/tert-butanol (v/v, 1/1) for 16 h to graft the dye molecules onto the TiO₂ surface. The counter electrodes consisted of PEDOT films electrochemically deposited on FTO glass. The dye-coated TiO₂ working electrode and the counter electrode were assembled by using thermoplastic spacer (Surlyn, DuPont) heating at 120°C. Electrolytes were injected into the space between the electrodes through predrilled hole on the counter electrode.

The Electrolyte I contains 0.25 M [Cu(tmbe)₂]TFSI complex melt, 0.05M NOBF₄, 0.1 M LiTFSI and 0.6 M 4-tert-butylpyridine (TBP) in acetonitrile. The NOBF₄ oxidizes the copper(I) complex melt to [Cu(tmbe)₂](TFSI)₂ melt in-situ.

The Electrolyte II contains 0.25 M [Cu(4,4',6,6'-tetramethyl-2,2'-bipyridine)₂](2-(2,5,8,11-tetraoxadodecanoyl)benzamide sulfonate), 0.05 M [Cu(4,4',6,6'-tetramethyl-2,2'-bipyridine)₂](2-(2,5,8,11-tetraoxadodecanoyl)benzamide sulfonate))₂, 0.6 M 4-tert-butylpyridine (TBP) and 0.1 M LiTFSI in acetonitrile. The DSCs based on copper complex melt hole transporting material were obtained by removing the solvent through the hole on the counter electrode.

The electrolyte III contains [Cu(4,4'-bis(2-(2-(2-methoxyethoxy)ethoxy)ethoxy)-6,6'-dimethyl-2,2'-bipyridine)₂](TFSI), [Cu(4,4'-bis(2-(2-(2-methoxyethoxy)ethoxy)ethoxy)-6,6'-dimethyl-2,2'-bipyridine)₂ (TFSI)₂ in a 5:1 molar ratio respectively, 0.6 M 4-tert-butylpyridine (TBP) and 0.1 M LiTFSI without any solvent.

### Characterization of solar cells

Solar cells were used without antireflection films and masked to an aperture area of 0.158 cm² for *J-V* and IPCE characterizations. *J-V* characteristics were recorded by a Keithley 2400 source meter. The solar cells were measured under irradiation intensity of 1,000 W/m² provided by a 450 W Xenon lamp of the Oriel solar simulator. The Oriel is equipped with a SchottK113 Tempax sunlight filter (Praezisions Glas & OptikGmbH) to match the emission spectrum of the lamp to the AM1.5G standard. The light intensity was determined using a calibrated Si reference diode equipped with an infrared cutoff filter (KG-3, Schott). IPCE spectra were measured with a lock-in amplifier (Stanford Research System SR830 DSP) under chopped monochromatic light (2 Hz) generated by white light source from a 300 W xenon lamp passing through a Gemini-180 double monochromator (Jobin Yvon Ltd). The solar cell is illuminated under a constant white light bias with intensity of 50 W/m² supplied by an array of white light emitting diodes.

### UV/Vis spectroscopy, steady-state photoluminescence spectroscopy, and TCSPC

UVNis absorption data was collected by a Perkin-Elmer Lambda 950 spectrophotometer and the extinction coefficients were calculated using the Beer-Lambert law. Steady-state photoluminescence spectra were recorded by exciting the samples at 450 nm with a 450-W Xenon CW lamp. The signal was recorded with a spectrofluorometer (Fluorolog; Horiba Jobin Yvon Technology FL1065). The time-correlated single photon counting (TSCPC) measurements were performed by exciting samples with a laser source at 408 nm (Horiba NanoLED 402-LH; pulse width <200 ps, 11 pJ per pulse, approximately 1mm² in spot size) to generate a train of excitation pulses at 10 MHz. Decay curves were analyzed with the software DAS-6 and DataStation provided by Horiba Jobin Yvon.

### Electrochemical Characterization

Three-electrode cyclic voltammetry measurements were performed using a Autolab Pgstat-30 potentiostat with Ag/AgCl/saturated LiCl (ethanol) as reference electrode and glassy carbon or platinum working electrodes under argon. Copper complexes were dissolved in acetonitrile, with LiTFSI (0.1M) as supporting electrolyte.

Here, we report a copper complex melt and its applications in DSCs. We characterized the copper complex melt with UV-visible spectroscopy, PL, and electrochemistry.

**Table 1: The photovoltaic performance under air mass 1.5 global with variant irradiation intensities of a DSC with copper(I/II) melt hole transporting materials in combination with Y123 dye, which was achieved by evaporating the acetonitrile from Electrolyte I as described in the fabrication of solar cells.**

| **Illumination intensity (mW/cm²)** | **Jsc (mA/cm²)** | **Voc (mV)** | **FF** | **PCE (%)** |
|---|---|---|---|---|
| 100.0 | 0.97 | 952 | 0.732 | 0.7 |
| 50.0 | 0.71 | 943 | 0.765 | 1.0 |
| 10.0 | 0.44 | 899 | 0.789 | 3.1 |

For DSCs based on the copper complex melt dissolved in acetonitrile (liquid Electrolyte I), we obtained PCE of 3.1% under 10% sun and 0.7% under full sun (standard AM1.5G conditions). When the acetonitrile solvent is evaporated, the photovoltaic parameters obtained were tabulated in Table 1.

**Table 2: The photovoltaic performance under air mass 1.5 global with variant irradiation intensities of a DSC with copper(I/II) melt hole transporting materials, which was achieved by evaporating the acetonitrile from Electrolyte II as described in the fabrication of solar cells.**

| **Illumination intensity (mW/cm²)** | **Jsc (mA/cm²)** | **Voc (mV)** | **FF** | **PCE (%)** |
|---|---|---|---|---|
| 100.0 | 7.66 | 948 | 0.611 | 4.4 |
| 50.0 | 5.93 | 932 | 0.676 | 7.5 |
| 10.0 | 1.19 | 872 | 0.775 | 8.0 |

The DSC based on the copper melt dissolved in acetonitrile (liquid electrolyte II) in combination with Y123 dye, we obtained PCE of 8.4% under 10% and full sun (standard AM1.5G conditions) intensities. When the acetonitrile solvent is evaporated, the photovoltaic parameters obtained were tabulated in Table 2.

**Table 3: The photovoltaic performance under air mass 1.5 global with variant irradiation intensities of a DSC with copper(I/II) melt hole transport materials with Electrolyte III (without any solvent) as described in the fabrication of solar cells.**

| **Illumination intensity (mW/cm²)** | **Jsc (mA/cm²)** | **Voc (mV)** | **FF** | **PCE (%)** |
|---|---|---|---|---|
| 100.0 | 1.3 | 836 | 0.382 | 0.42 |
| 50.0 | 0.969 | 969 | 0.372 | 0.45 |
| 10.0 | 0.825 | 765 | 0.361 | 1.87 |

The initial and un-optimized device photovoltaic parameters of devices fabricated with solvent free copper redox melt electrolyte in combination with Y123 dye is shown in Table 3. The device performance will be enhanced by designing small size copper redox melts and decreasing the viscosity of the redox electrolytes. To increase the device performance it is necessary to optimize the pore size of the TiO₂ to facilitate the diffusion of viscous redox melt electrolyte into the TiO₂ pores. It is important to use solvent free electrolyte in DSC to avoid the leakage of electrolyte from the device and enhance the durability of devices.

## Claims

1. A redox copper complex melt having a melting point below 100°C and comprising a metalorganic complex of formula (I)
[Cu (La)ₙ] (I),
wherein
n is an integer 2; and
La is a bidentate ligand selected from a ligand according to any one of formulae (1) and (2): 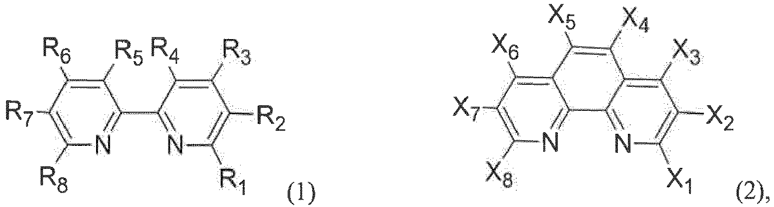 wherein:
R₁, R₂, R₃, R₄, R₅, R₆, R₇, R₈, X₁, X₂, X₃, X₄, X₅, X₆, X₇, and X₈ are independently selected from H, halogen, Cl⁻, Br⁻, I⁻,-NO₂, -OH, -NH₂, -COOH, -CN, -OCN⁻, isocyanate group, sulfonyl group and from substituents comprising 1 to 40 carbons and 0 to 20 heteroatoms and/or groups selected from C1-C25 alkyl group, C4-C20 aryl group, C4-C20 arylalkyl group, C1-C25 alkoxy group, C4-C25 alkoxyalkoxyalkoxyalkyl group, C1-C20 heteroalkyl group, C4-C20 heteroaryl group, C4-C20 heteroarylalkyl group, said heteroatom being selected from N, S, or O, and from -O-, -O-R⁶, -O-R⁶-R⁶,-C(O)-O-R⁶, -C(O)-(NR⁷)-O-R⁶, -C(O)-(NR⁷)-R⁶; with R⁶ being independently selected from C1-C25 alkyl group or 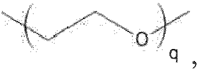 q being an integer from 1 to 20, and with R⁷ being selected from H or C1-C16 alkyl group, from - (CH₂)₂-O-, -C(O)-, -C(O)O-, -S-, -S(O)-, SO₂-, -S(O)₂O-, -N=, -P=, -NR⁹-,-PR⁹-, -P(O)(OR⁹)-, -P(O)(OR⁹)O-, -P(O)(NR⁹R⁹)-, -P(O)(NR⁹R⁹)O-,-P(O)(NR⁹R⁹)NR⁹-, -S(O)NR⁹-, and -S(O)₂NR⁹, with R⁹ being independently selected from H, C1-C6 alkyl, C4-C20 aryl group, C4-C20 arylalkyl group and C4-C20 heteroaryl group, wherein the heteroatom is selected from N, S, or O, and said alkyl, arylalkyl and heteroaryl groups being optionally perfluorinated.

2. The redox copper complex melt according to claim 1, wherein the metalorganic complex of formula (I) is cationic and the redox metal complex melt further comprises a negative counterion selected from ClO₄⁻, PF₆⁻, BF₄⁻, [B(CN)₄]⁻, CF3SO₃⁻, I(CF₃SO₂)₂N]⁻ (TFSI), [B(C₆H₃(m-CF₃)₂)4]⁻, [B(C₆F₅)₄]⁻, [B(C₆H₅)₄]⁻, [Al(OC(CF₃)₃)₄]⁻, [CB₁₁H₁₂]⁻, and a moiety according to formula (3) 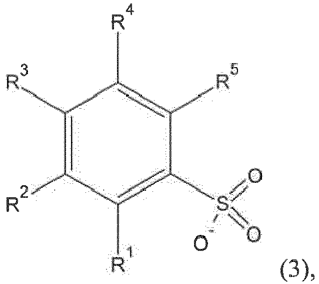 wherein R¹, R², R³, R⁴, R⁵ are independently selected from H, -CF₃ and substituents comprising 1 to 40 carbons and 0 to 20 groups selected from C1-C16 alkyl group, C1-C25 alkoxy group, C4-C25 alkoxyalkoxyalkoxyalkyl group, C4-C20 aryl group, C4-C20 arylalkyl group, -O-, -CF₃, -O-R⁶, -O-R⁶-R⁶,-C(O)-O-R⁶, -C(O)-(NR⁷)-OR⁶, -C(O)-(NR⁷)-R⁶, with R⁶ being independently selected from C1-C25 alkyl group or 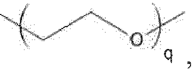 q being an integer from 1 to 20, and with R⁷ being selected from H or C1-C16 alkyl group; or from a salt of a moiety of formula (3).

3. The redox copper complex melt according to claim 2, wherein the moiety of formula (3) is selected from a moiety according to anyone of formulae (4) to (8) 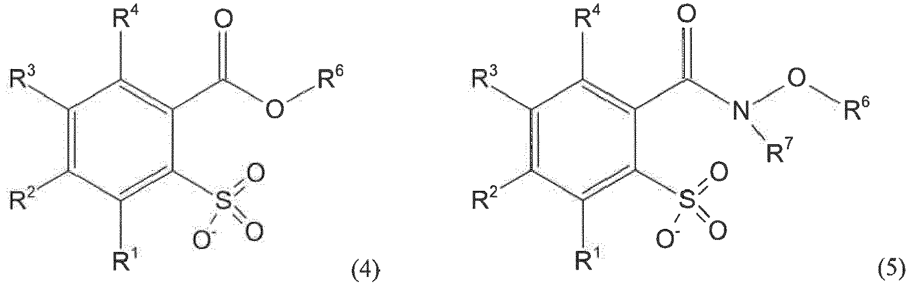 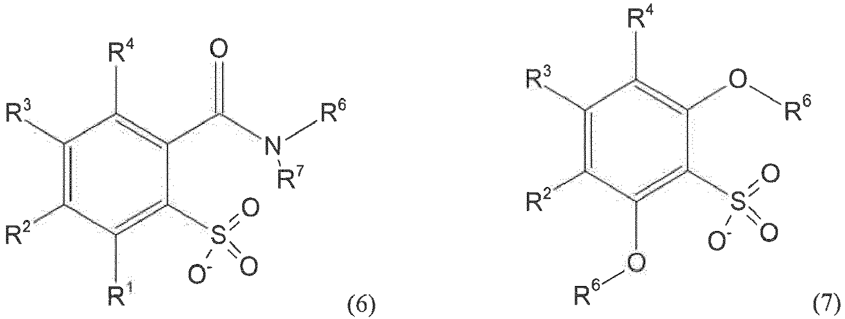 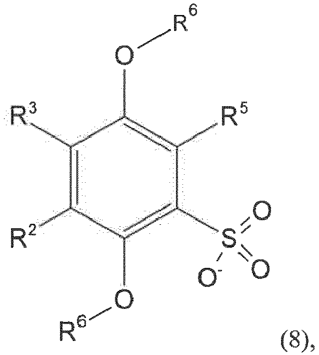 wherein R¹, R², R³, R⁴, R⁵, R⁶ and R⁷, if present, are defined as in claim 2.

4. The redox copper complex melt according to any one of the preceding claims, wherein R₂, R₄, R₅ and R₇ of La ligand of formula (1) and/or X₂, X₄, X₅, and X₇ of La ligand of formula (2) are H.

5. The redox copper complex melt according to any one of the preceding claims, wherein R₁ and R₈ of La ligand of formula (1) and/or X₁ and X₈ of La ligand of formula (2) are selected from H or substituents comprising 1 to 40 carbons and 0 to 20 groups selected from C1-C25 alkyl group, C4-C20 aryl group, C4-C20 arylalkyl group.

6. The redox copper complex melt according to any one of the preceding claims, wherein R₃ and R₆ of La ligand of formula (1) and/or X₃ and X₆ of La ligand of formula (2) are selected from H or from substituents comprising 1 to 40 carbons and 1 to 20 heteroatoms and/or groups selected from C1-C25 alkyl group, C4-C20 aryl group, C4-C20 arylalkyl group, C1-C25 alkoxy group, C4-C25 alkoxyalkoxyalkoxyalkyl group, C1-C20 heteroalkyl group, C4-C20 heteroaryl group, C4-C20 heteroarylalkyl group, said heteroatom being selected from N, S, or O, and from -O-, -O-R⁶, -O-R⁶-R⁶,-C(O)-O-R⁶, -C(O)-(NR⁷)-O-R⁶,-C(O)-(NR⁷)-R⁶; with R⁶ being independently selected from C1-C25 alkyl group or 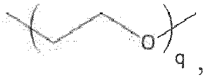 q being an integer from 1 to 20, and with R⁷ being selected from H or C1-C16 alkyl group, from -(CH₂)₂-O-, -C(O)-, -C(O)O-, and -NR⁹-, with R⁹ being independently selected from H, C1-C6 alkyl, C4-C20 aryl group, C4-C20 arylalkyl group and C4-C20 heteroaryl group, wherein the heteroatom is selected from N, S, or O.

7. The redox copper complex melt according to any one of the preceding claims, wherein R₃ and R₆ of La ligand of formula (1) and/or X₃ and X₆ of La ligand of formula (2) are selected from R₃ and R₆ of La ligand of formula (1) and/or X₃ and X₆ of La ligand of formula (2) may be further selected from H or substituents comprising 1 to 40 carbons and 0 to 20 groups selected from C1-C25 alkyl group, C1-C25 alkoxy group, C2-C25 alkoxyalkyl group, C4-C20 aryl group, C4-C20 arylalkyl group, C4-C25 alkoxyaryl group, C5-C25 alkoxyarylalkyl group, C6-C25 alkoxyarylalkoxyalkyl group.

8. The redox copper complex melt according to any one of claims 2-3, wherein the negative counter ion is provided from a salt of moiety (3) or a salt according to any one of moieties of formulae (4) to (8), wherein said salt comprises a cation selected from Na⁺, Li⁺, Cs⁺ and K⁺.

9. The redox copper complex melt according to any one of the preceding claims, wherein R₃ and R₆ of La ligand of formula (1) and/or X₃ and X₆ of La ligand of formula (2) are selected from substituents comprising 1 to 20 groups selected from C1-C25 alkoxy group, C4-C25 alkoxyalkoxyalkoyalkyl group, C3-C25 alkoxyalkoxyalkyl group, methoxyethanyl group, 1-methoxy-2-[2-(2-methoxyethoxy)ethoxy]ethanyl group, 1-methoxy-2-(2-methoxyethoxy)ethanyl group, 1,2-dimethoxyethanyl group or 2-(2-(2-methoxyethoxy)ethoxy)ethanyl group.

10. The redox copper complex melt according to any one of the preceding claims, wherein La of formula (1) is 4,4'-bis(2-(2-(2-methoxyethoxy)ethoxy)ethoxy)-6,6'-dimethyl-2,2'-bipyridine and/or La of formula (2) is 4,7-bis(2-(2-(2-methoxyethoxy)ethoxy)ethoxy)-2,9-dimethyl-1,10-phenanthroline.

11. An optoelectronic and/or electrochemical device or redox flow batteries comprising at least one or more redox coper complex melt according to any one of claims 1-10.

12. The optoelectronic and/or electrochemical device or redox flow batteries according to claim 11, wherein the redox copper complex melt is one of the components of electrolyte or hole transporting material.

13. The optoelectronic and/or electrochemical device according to any one of claims 11-12 being selected from a photoelectric conversion device, a photovoltaic cell or a dye-sensitized solar cell.

14. A method for producing a redox copper complex melt comprising a metalorganic complex of formula (I):
- providing a solution comprising ligand La selected from formula (1) or from formula (2) as defined in claim 1, and further comprising a salt of copper;
- exchanging the anion provided by the copper salt by titration with a solution comprising an anion ClO₄⁻, PF₆⁻, BF₄⁻, [B(CN)₄]⁻, CF3SO₃⁻, [(CF₃SO₂)₂N]⁻ (TFSI), [B(C₆H₃(m-CF₃)₂)₄]⁻, [B(C₆F₅)₄]⁻, [B(C₆H₅)₄]⁻, [Al(OC(CF₃)₃)₄]⁻, [CB₁₁H₁₂]⁻, a moiety according to formula (3) 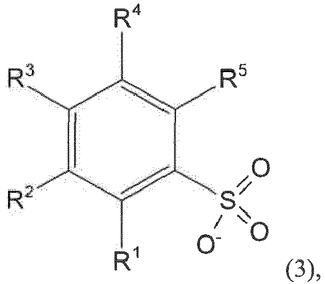 wherein R¹, R², R³, R⁴, R⁵ are independently selected from H and substituents comprising 1 to 40 carbons and 0 to 20 groups selected from C1-C16 alkyl group, C1-C25 alkoxy group, C4-C25 alkoxyalkoxyalkoxyalkyl group, C4-C20 aryl group, C4-C20 arylalkyl group, -O-, -CF₃, -O-R⁶, -O-R⁶-R⁶,-C(O)-O-R⁶, -C(O)-(NR⁷)-OR⁶, -C(O)-(NR⁷)-R⁶, with R⁶ being independently selected from C1-C25 alkyl group or 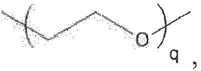 q being an integer from 1 to 20, and with R⁷ being selected from H or C1-C16 alkyl group; or a salt of a moiety of formula (3); and
- evaporating the titrated crude solution to obtain a melt comprising the redox copper complex melt.
